# EUROPEAN PATENT APPLICATION

(11) **EP 0 877 246 A2**
(43) Date of publication of application: **11.11.1998**
(21) Application number: 98301789.8
(22) Date of filing: 11.03.1998
(51) Int. Cl.: G01N 33/00, G01N 27/00

(54) **In situ monitoring of contaminants in semiconductor processing chambers**

(30) Priority: 11.03.1997 US 814565
(71) Applicant: Applied Materials, Inc., Santa Clara, California 95052 (US)
(72) Inventor: Hashim, Imran, Fremont, California 94538 (US)
(74) Representative: Bayliss, Geoffrey Cyril

(57) **Abstract**

A method and apparatus for measuring essentially real-time concentrations of gaseous contaminants exiting a semiconductor processing chamber (10). A small volume of exhaust gases from the processing chamber (10) is drawn through one or more microsensors (56). The microsensors (56) are selected to measure gaseous contaminants such as moisture, oxygen, carbon dioxide, or combinations thereof. *In situ* monitors comprising a thin film sensor are preferred. The *in situ* monitors preferably can be selectively communicated with the exhaust gases from the processing chamber (10) and process gases which bypass the processing chamber (10).

## Description

The present invention relates generally to devices and methods for measuring contaminants in a process stream. More particularly, the present invention relates to devices and methods for measuring contaminants in processing chambers used to make semiconductors.

A device currently used to measure contaminants in a semiconductor processing chamber is a residual gas analyzer (RGA) which preferably includes quadropole technology. For monitoring of contaminants at processing pressures, a dedicated turbo pump draws chamber gases through a control valve or a limiting orifice into the analyzer. Such a residual gas analyzer is bulky and expensive, and is generally incapable of monitoring contaminants in process gases prior to their entry into the chamber.

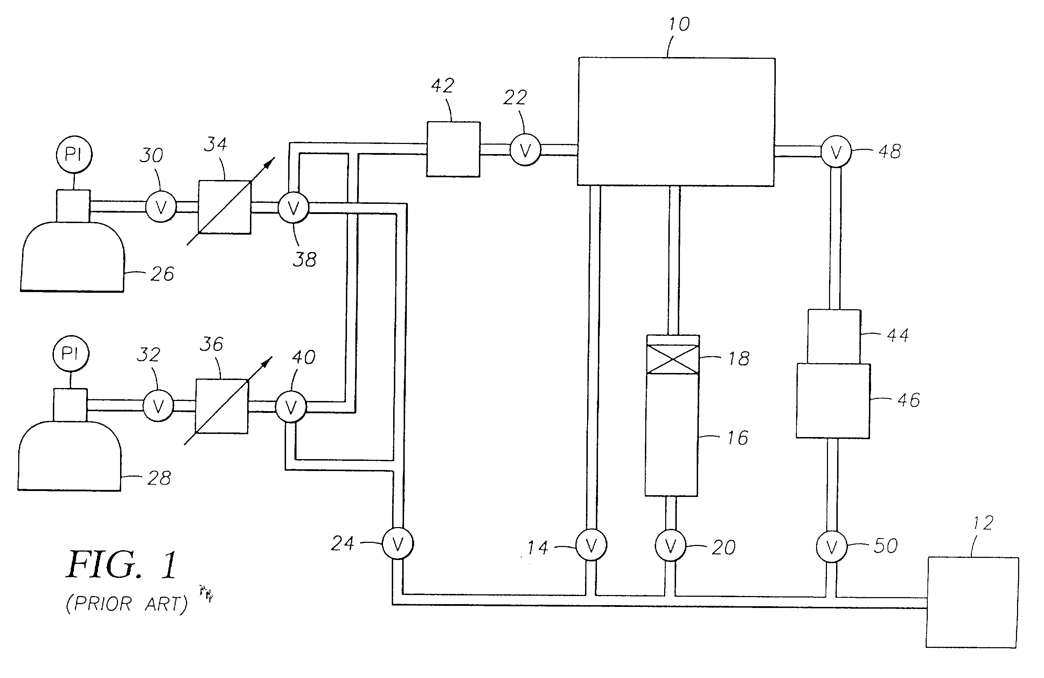

Figure 1 (Prior Art) is a schematic diagram ofa semiconductor processing chamber 10 which is initially evacuated by a roughing pump 12 through an open shut-off valve 14. A high vacuum pump 16, such as a cryogenic pump, then evacuates the chamber 10 through a control gate valve 18 by closing the shut-off valve 14 and opening a second shut-off valve 20. The high vacuum pump 16 continues to pump the chamber 10 during processing of semiconductor wafers. One or more processing gases (two shown) are selectively provided to the processing chamber 10 through a final inlet valve 22 or directly to the roughing pump 12 through a bypass valve 24. The process gases are typically supplied from gas cylinders 26, 28 through cylinder shut-off valves 30, 32. The flow rate ofthe gases is typically controlled with mass flow controllers 34, 36. Three way valves 38, 40 can be used to selectively flow the process gases to the chamber 10 or to the roughing pump 12. Each three way valve may be replaced by a tee and two shut-off valves positioned on the outlets.

An *in situ* moisture analyzer 42 may be used to monitor the process gases prior to entering the chamber 10. A preferred moisture analyzer has a thin film of barium coated on a quartz crystal microbalance sensor and is commercially available from Millipore Corporation under the trademark ILM®. United States Patents Nos. 5,339,675 and 5,477,716 describe thin film sensors for monitoring moisture in process gasses which are inlet to semiconductor processing chambers.

A residual gas analyzer 44 measures contaminants within the processing chamber 10. Contaminants typically of concern include moisture, oxygen, and carbon dioxide. A dedicated turbo pump 46 draws a small volume of gases from the chamber 10 through a control valve 48, or a limiting orifice. Sampling of the gases in the chamber 10 is selected by a shut-off valve which connects the turbo pump 46 and the roughing pump 12.

The residual gas analyzer 44 does not operate at increased pressures such as the regimes typically found in process gas lines which supply the processing chamber 10. Additional monitors for process gases are typically used to monitor contaminants in the process gases. Therefore, there exists a need for an apparatus and method for measuring contaminants in either process gasses or in a processing chamber.

The present invention provides a method and apparatus for essentially *in situ* monitoring of contaminants within a semiconductor processing chamber. According to a method and apparatus of one embodiment of the present invention, a small volume of exhaust gases are drawn through one or more micro sensors by a dedicated smaller high vacuum pump. The microsensors are selected to measure gaseous contaminants such as moisture, oxygen, carbon dioxide, or combinations thereof. *In situ* monitors comprising a thin film sensor are preferred. The *in situ* monitors are readily configured to select measurement of contaminants in process gases or in the processing chamber.

So that the manner in which the above recited features, advantages and objects of the present invention are attained and can be understood in detail, a more particular description of the invention, briefly summarized above, may be had by reference to the embodiments thereof which are illustrated in the appended drawings.

It is to be noted, however, that the appended drawings illustrate only typical embodiments of this invention and are therefore not to be considered limiting of its scope, for the invention may admit to other equally effective embodiments.
Figure 1 (Prior Art) is a schematic view of a semiconductor processing chamber having a microsensor measuring the moisture content of inlet gases and a residual gas analyzer measuring gaseous contaminants exiting the processing chamber;
Figure 2 is a schematic view of the semiconductor processing chamber having a microsensor which selectively communicates with the inlet gases and exhaust gases to measure gaseous contaminants entering and exiting the processing chamber; and
Figure 3 is an alternative schematic view of the semiconductor processing chamber and the microsensor of Figure 2.

For a further understanding of the present invention, reference should be made to the ensuing detailed description.

In a preferred embodiment, the present invention provides an apparatus for measuring contaminants in a semiconductor processing chamber, the apparatus comprising a processing chamber, and one or more microsensors, wherein the microsensors are positioned to receive exhaust gases from the processing chamber and measure essentially real-time concentrations of one or more contaminants. A small volume of exhaust gases are drawn through one or more microsensors by a dedicated smaller high vacuum pump. The microsensors are selected to measure gaseous contaminants such as moisture, oxygen, carbon dioxide, or combinations thereof. *In situ* monitors comprising a thin film sensor are preferred.

The apparatus of the invention preferably comprises a processing chamber comprising a gas inlet and an exhaust port, a source of one or more process gases connected to the gas inlet, a high vacuum pump connected to the exhaust port, one or more *in situ* monitors, and one or more valves positioned to selectively communicate the *in situ* monitors with the source of one or more process gases and a portion of gases exhausted from the processing chamber.

In another preferred embodiment, the present invention provides a method for measuring contaminants in a semiconductor processing chamber, comprising the steps of flowing exhaust gases from the processing chamber through one or more microsensors, and measuring essentially real-time concentrations of one or more contaminants. The method of the invention preferably comprises the combined steps of evacuating the processing chamber with a high vacuum pump, and selectively communicating one or more *in situ* monitors with exhaust gases flowing from the processing chamber and process gases bypassing the processing chamber.

The method and apparatus can be used with any processing chamber, and is ideally suited for the low pressures and high temperatures encountered in physical vapor deposition of metal layers on semiconductor wafers.

Further description of the invention will be directed toward specific embodiments.

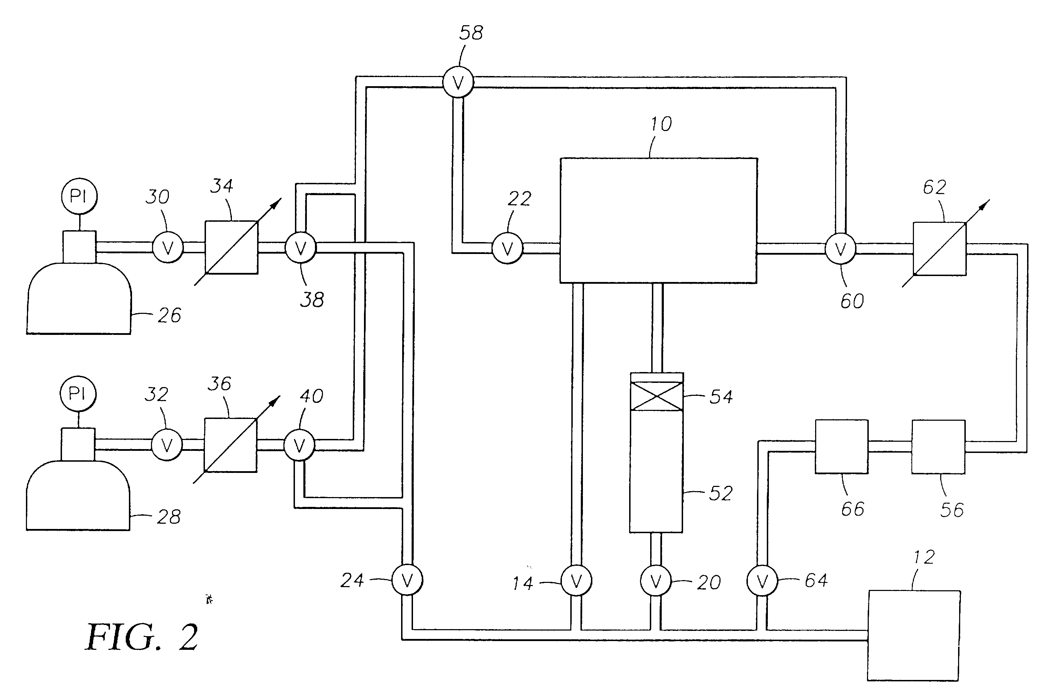

Figure 2 is a schematic diagram of a semiconductor processing chamber 10 which is initially evacuated by a roughing pump 12 through an open shutoff valve 14. A high vacuum pump 52, such as a cryogenic pump, then evacuates the chamber 10 through a control gate valve 54 by closing the shut-off valve 14 and opening a second shut-off valve 20. The high vacuum pump 16 continues to pump the chamber 10 during processing of semiconductor wafers. One or more source gases (two shown) are selectively provided to the processing chamber 10 through a final inlet valve 22 or directly to the roughing pump 12 through a bypass valve 24. The process gases are typically supplied from gas cylinders 26, 28 through cylinder shut-off valves 30, 32. The flow rate of the gases is typically controlled with mass flow controllers 34, 36. Three way valves 38, 40 are used to selectively flow the process gases to the chamber 10 or to the roughing pump 12. Each three way valve may be replaced by a tee and two shut-off valves positioned on the tee outlets.

A microsensor moisture analyzer 56 is positioned to selectively communicate with the source of process gases or a portion of exhaust gases from the chamber 10 through a first three-way valve 58 and a second three-way valve 60. The process or exhaust gases are drawn through the microsensor by a dedicated pump 66, and the flow rate is controlled by a needle valve 62, or an orifice having adjustable conductance. A shutoff valve 64 isolates the microsensor 56 from the roughing pump 12 when desired. Alternatively, the shut-off valve could isolate the microsensor 56 from the high vacuum pump, and a small turbomolecular pump or a drag pump would be sufficient to draw the process gas or the chamber exhaust through the monitor.

Microsensors for other gaseous contaminants can supplement or replace the moisture analyzer 56. Preferred *in situ* monitors have a thin film sensor which absorbs the gaseous contaminant. A preferred moisture analyzer has a thin film of barium coated on a quartz crystal microbalance sensor and is commercially available from Millipore Corporation. Monitors for other gaseous contaminants include Titanium based microsensors for monitoring Oxygen.

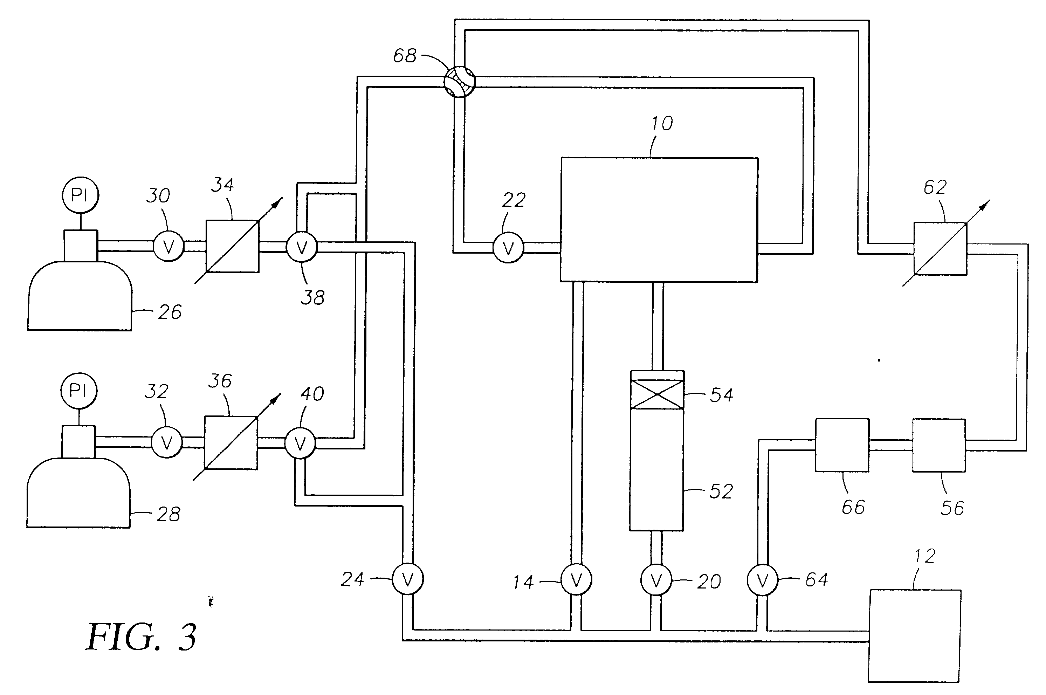

Figure 3 is a schematic diagram of the semiconductor processing chamber 10 and the microsensor 56 of Figure 2 wherein the first and second three-way valves 58, 60 are replaced by a four-way valve 68 which selectively communicates the microsensor 56 with the source of process gases and a portion of exhaust gases from the chamber.

The microsensor 56 preferably measures contaminants exiting the processing chamber 10 during processing of wafers after the processing chamber is evacuated by the roughing pump 12 and the high vacuum pump 52. Contaminants in the inlet gases may be monitored as desired after the inlet gas lines are evacuated by the roughing pump 12. During wafer processing, monitoring of inlet process gases and chamber exhaust gases is selected by simultaneously switching the first and the second three-way valves 58, 60 (Fig. 2) or the four-way valve 68 (Fig. 3).

## Claims

1. A method for measuring contaminants in a semiconductor processing chamber, comprising the steps of:
flowing exhaust gases from the processing chamber through one or more microsensors; and
measuring essentially real-time concentrations of one or more contaminants.

2. The method of claim 1, wherein at least one microsensor comprises a thin film sensor.

3. The method of claim 1, wherein at least one microsensor is an *in situ* monitor which comprises a quartz crystal coated with barium.

4. The method of claim 1, wherein a dedicated pump draws exhaust gases from the processing chamber through the microsensor.

5. The method of claim 1, further comprising the step of selectively communicating the microsensor with the exhaust gases flowing from the processing chamber.

6. A method for measuring one or more gaseous contaminants in a semiconductor processing chamber, comprising the steps of:
evacuating the processing chamber with a high vacuum pump; and
selectively communicating one or more *in situ* monitors with exhaust gases flowing from the processing chamber and process gases bypassing the processing chamber.

7. The method of claim 6, wherein at least one *in situ* monitor comprises a thin film sensor.

8. The method of claim 6, wherein at least one *in situ* monitor comprises a quartz crystal coated with barium.

9. The method of claim 6, wherein the real-time concentration of water is continuously measured.

10. The method of claim 6, wherein the processing chamber is a PVD processing chamber.

11. An apparatus for measuring contaminants in a semiconductor processing chamber, comprising:
a processing chamber; and
one or more microsensors, at least one micosensor positioned to receive exhaust gases from the processing chamber and measure essentially real-time concentrations of one or more contaminants.

12. The apparatus of claim 11, wherein at least one microsensor comprises a thin film sensor.

13. The apparatus of claim 11, wherein at least one microsensor is an *in situ* monitor which comprises a quartz crystal coated with barium.

14. The apparatus of claim 11, wherein a dedicated pump draws a portion of the exhaust gases from the processing chamber through the microsensor.

15. The apparatus of claim 11, further comprising one or more valves for selectively communicating the microsensor with the exhaust gases.

16. An apparatus for measuring contaminants in a semiconductor processing chamber, comprising;
a processing chamber comprising a gas inlet and an exhaust port;
a source of one or more process gases connected to the gas inlet;
a high vacuum pump connected to the exhaust port;
one or more *in situ* monitors; and
one or more valves positioned to selectively communicate the *in situ* monitors with the source of one or more process gases and a portion of gases exhausted from the processing chamber.

17. The apparatus of claim 16, wherein at least one *in situ* monitor comprises a thin film sensor.

18. The apparatus of claim 16, wherein at least one *in situ* monitor comprises a quartz crystal coated with barium.

19. The apparatus of claim 16, wherein at least one *in situ* monitor measures essentially real-time concentrations of moisture.

20. The apparatus of claim 16, wherein the processing chamber is a PVD processing chamber.
